# EUROPEAN PATENT APPLICATION

(11) **EP 4 512 308 A1**
(43) Date of publication of application: **26.02.2025**
(21) Application number: 23792087.1
(22) Date of filing: 11.04.2023
(51) Int. Cl.: A61B 5/00, G06T 7/30

(54) **ELECTRONIC APPARATUS, METHOD, AND RECORDING MEDIUM FOR GENERATING AND ALIGNING THREE-DIMENSIONAL IMAGE MODEL OF THREE-DIMENSIONAL SCANNER**

(30) Priority: 20.04.2022 KR 20220048871
(71) Applicant: Medit Corp., Seoul 07207 (KR)
(72) Inventor: KO, Ki Nam, Seoul 07207 (KR)
(74) Representative: MFG Patentanwälte Meyer-Wildhagen Meggle-Freund Gerhard PartG mbB
(86) International application number: PCT/KR2023/004843
(87) International publication number: WO 2023/204509

(57) **Abstract**

The present disclosure relates to an apparatus, a method, and non-transitory computer-readable recording medium storing program for generating and aligning a three-dimensional image model of a three-dimensional scanner. The apparatus according to one embodiment of the present disclosure may: obtain first scan data values for a surface of an object by first scanning by the three-dimensional scanner, the first scan data values including three-dimensional coordinate values, generate a first three-dimensional image model based on the first scan data values, obtain second scan data values for the surface of the object by second scanning by the three-dimensional scanner performed after generation of the first three-dimensional image model, the second scan data values including three-dimensional coordinate values, and generate a second three-dimensional image model by automatically replacing at least a part of the first scan data values with the second scan data values or based on the second scan data values independently of the first three-dimensional image model.

## Description

### TECHNICAL FIELD

The present disclosure relates to a technique for generating and aligning three-dimensional image models of an electronic apparatus and a three-dimensional scanner, and, more particularly, to a technique for generating a subsequent three-dimensional image model of an object or aligning a subsequent three-dimensional image model with an existing three-dimensional image model.

### BACKGROUND

A three-dimensional intraoral scanner is an optical device that is inserted into a patient's oral cavity and scans teeth to obtain a three-dimensional image of the oral cavity. By scanning the patient's oral cavity using a three-dimensional scanner, a plurality of two-dimensional images of the patient's oral cavity may be obtained, and a three-dimensional image of the patient's oral cavity may be constructed using the plurality of two-dimensional images obtained. For example, a doctor may insert the three-dimensional scanner into the patient's oral cavity and scan the patient's teeth, gum, and/or soft tissue to obtain a plurality of two-dimensional images of the patient's oral cavity. Then, by applying a three-dimensional modeling technique, a three-dimensional image of the patient's oral cavity may be constructed using the two-dimensional images of the patient's oral cavity.

If various treatments are performed on the patient's oral cavity after performing three-dimensional scanning for the patient's oral cavity, which brings about a change in the teeth or the like, the three-dimensional scanning may need to be reperformed on the patient's oral cavity. For example, when a patient's tooth is partially cut and a prosthesis manufactured to fit the shape of the cut tooth is inserted, three-dimensional scanning for the oral cavity in the initial state before cutting the patient's tooth, three-dimensional scanning for the oral cavity after cutting the patient's tooth, and three-dimensional scanning for the oral cavity after inserting the prosthesis may be performed. In this case, conventionally, subsequent three-dimensional scanning must be performed from the beginning, or the subsequent three-dimensional scanning must include scanning for a portion overlapping the existing three-dimensional data, which may be cumbersome and difficult. In particular, if the size of the area that has been changed in the existing tooth (i.e., where the tooth has been cut or a prosthesis has been inserted into the tooth) is large, a portion overlapping the existing three-dimensional data becomes small, making it difficult to acquire data of the overlapping portion, which may lead to failure of subsequent three-dimensional scanning. In addition, when subsequent three-dimensional scanning is newly performed from the beginning, it is necessary to align the existing three-dimensional image and the subsequent three-dimensional image in order to compare the state of the oral cavity before and after the treatment.

### SUMMARY

A technical problem to be solved through an embodiment of the present disclosure is to process a scan image obtained by a three-dimensional scanner.

Another technical problem to be solved through an embodiment of the present disclosure is to improve inconvenience in a subsequent three-dimensional scanning process, thereby enhancing user experience.

Another technical problem to be solved through an embodiment of the present disclosure is to provide a method for aligning and comparing three-dimensional images respectively obtained through three-dimensional scanning.

The technical problems of the present disclosure are not limited to the technical problems mentioned above, and other technical problems not mentioned will be clearly understood by those skilled in the art of the present disclosure from the description below.

According to one embodiment of the present disclosure, an electronic apparatus may comprise: a communication circuit in communication connection with a three-dimensional scanner; one or more processors; and one or more memories storing instructions executed by the one or more processors, wherein, when the instructions are executed by the one or more processors, the one or more processors: obtain first scan data values for a surface of an object by first scanning by the three-dimensional scanner, the first scan data values including three-dimensional coordinate values; generate a first three-dimensional image model based on the first scan data values; obtain second scan data values for the surface of the object by second scanning by the three-dimensional scanner performed after generation of the first three-dimensional image model, the second scan data values including three-dimensional coordinate values; and generate a second three-dimensional image model by automatically replacing at least a part of the first scan data values with the second scan data values or based on the second scan data values independently of the first three-dimensional image model.

In one embodiment, the generating of the second three-dimensional image model by automatically replacing at least a part of the first scan data values with the second scan data values may include: comparing the first scan data values with the second scan data values to determine first sub-data values that are at least a part of the first scan data values and second sub-data values that are at least a part of the second scan data values; and generating the second three-dimensional image model based on third scan data values obtained by replacing the first sub-data values of the first scan data values with the second sub-data values.

In one embodiment, the determining of the first sub-data values and the second sub-data value may include: determining first vectors connecting a virtual focus of the three-dimensional scanner and the first scan data values or second vectors connecting the virtual focus and the second scan data values; determining whether or not the first vectors intersect the second scan data values or whether or not the second vectors intersect the first scan data values; when at least one first vector among the first vectors intersects at least one second scan data value among the second scan data values, determining data values of the first scan data values, which are associated with the at least one first vector intersecting the at least one second scan data value, as the first sub-data values and determining data values of the second scan data values, which intersect the at least one first vector, as the second sub-data values; and when at least one second vector among the second vectors intersects at least one first scan data value among the first scan data values, determining data values of the first scan data values, which intersect the at least one second vector, as the first sub-data values and determining data values of the second scan data values, which are associated with the at least one second vector intersecting the at least one first scan data value, as the second sub-data values.

In one embodiment, the one or more processors may be configured to, after generating the second three-dimensional image model based on the second scan data value: obtain data indicating at least three points included in the first three-dimensional image model and data indicating at least three points included in the second three-dimensional image model; and align the first three-dimensional image model or the second three-dimensional image model based on the at least three points included in the first three-dimensional image model and the at least three points included in the second three-dimensional image model.

In one embodiment, the electronic apparatus may further comprise a display, and the one or more processors may be configured to: display the first three-dimensional image model and the second three-dimensional image model on the display; obtain inputs of data indicating at least three teeth included in the first three-dimensional image model and data indicating at least three teeth included in the second three-dimensional image model based on the first three-dimensional image model and the second three-dimensional image model displayed on the display; and determine three points respectively corresponding to the at least three teeth included in the first three-dimensional image model and three points respectively corresponding to the at least three teeth included in the second three-dimensional image model.

In one embodiment, the electronic apparatus may further comprise a display, and the one or more processors may be configured to: generate a first synthetic image model in which the first three-dimensional image model and the second three-dimensional image model, which have been aligned, are superimposed; and display the first synthetic image model on the display.

In one embodiment, the one or more processors may be configured to process the first three-dimensional image model to be transparent or translucent in the first synthetic image model.

According to one embodiment of the present disclosure, a method for processing a scan image obtained by a three-dimensional scanner performed by an electronic apparatus including one or more processors and one or more memories storing instructions to be executed by the one or more processors may comprise: obtaining first scan data values for a surface of an object by first scanning by the three-dimensional scanner, the first scan data values comprising three-dimensional coordinate values; generating a first three-dimensional image model based on the first scan data values; obtaining second scan data values for the surface of the object by second scanning by the three-dimensional scanner performed after generation of the first three-dimensional image model, the second scan data values comprising three-dimensional coordinate values; and generating a second three-dimensional image model by automatically replacing at least a part of the first scan data values with the second scan data values or based on the second scan data values, independently of the first three-dimensional image model.

In one embodiment, the generating of the second three-dimensional image model by automatically replacing at least a part of the first scan data values with the second scan data values may include: comparing the first scan data values with the second scan data values to determine first sub-data values that are at least a part of the first scan data values and second sub-data values that are at least a part of the second scan data values; and generating the second three-dimensional image model based on third scan data values obtained by replacing the first sub-data values of the first scan data values with the second sub-data values.

In one embodiment, the determining of the first sub-data values and the second sub-data value may include: determining first vectors connecting a virtual focus of the three-dimensional scanner and the first scan data values or second vectors connecting the virtual focus and the second scan data values; determining whether or not the first vectors intersect the second scan data values or whether or not the second vectors intersect the first scan data values; when at least one first vector among the first vectors intersects at least one second scan data value among the second scan data values, determining data values of the first scan data values, which are associated with the at least one first vector intersecting the at least one second scan data value, as the first sub-data values and determining data values of the second scan data values, which intersect the at least one first vector, as the second sub-data values; and when at least one second vector among the second vectors intersects at least one first scan data value among the first scan data values, determining data values of the first scan data values, which intersect the at least one second vector, as the first sub-data values and determining data values of the second scan data values, which are associated with the at least one second vector intersecting the at least one first scan data value, as the second sub-data values.

In one embodiment, the method may further comprise, after generating the second three-dimensional image model based on the second scan data value, independently of the first three-dimensional image model: obtaining data indicating at least three points included in the first three-dimensional image model and data indicating at least three points included in the second three-dimensional image model; and aligning the first three-dimensional image model or the second three-dimensional image model based on the at least three points included in the first three-dimensional image model and the at least three points included in the second three-dimensional image model.

In one embodiment, the obtaining of data indicating at least three points included in the first three-dimensional image model and data indicating at least three points included in the second three-dimensional image model may include: displaying the first three-dimensional image model and the second three-dimensional image model on a display; obtaining inputs of data indicating at least three teeth included in the first three-dimensional image model and data indicating at least three teeth included in the second three-dimensional image model based on the first three-dimensional image model and the second three-dimensional image model displayed on the display; and determining three points respectively corresponding to the at least three teeth included in the first three-dimensional image model and three points respectively corresponding to the at least three teeth included in the second three-dimensional image model.

In one embodiment, the method may further comprise: generating a first synthetic image model in which the first three-dimensional image model and the second three-dimensional image model, which have been aligned, are superimposed; and displaying the first synthetic image model on a display.

In one embodiment, the method may further comprise processing the first three-dimensional image model to be transparent or translucent in the first synthetic image model.

According to one embodiment of the present disclosure, in a non-transitory computer-readable recording medium storing instructions executable by one or more processors, when executed by the one or more processors, the instructions may cause the one or more processors to: obtain first scan data values for a surface of an object by first scanning by the three-dimensional scanner, the first scan data values comprising three-dimensional coordinate values; generate a first three-dimensional image model based on the first scan data values; obtain second scan data values for the surface of the object by second scanning by the three-dimensional scanner performed after generation of the first three-dimensional image model, the second scan data values comprising three-dimensional coordinate values; and generate a second three-dimensional image model by automatically replacing at least a part of the first scan data values with the second scan data values or based on the second scan data values, independently of the first three-dimensional image model.

According to the present disclosure, it is possible to process scan images obtained by a three-dimensional scanner.

According to the present disclosure, it is possible to improve inconvenience in a subsequent three-dimensional scanning process, thereby enhancing user experience.

According to the present disclosure, it is possible to provide a method for aligning and comparing three-dimensional images respectively obtained by three-dimensional scanning.

The effects according to the technical idea of the present disclosure are not limited to the effects mentioned above, and other effects not mentioned will be clearly understood by those skilled in the art from the description of the specification.

### BRIEF DESCRIPTION OF DRAWINGS

FIG. 1 is a diagram illustrating the state in which an image of a patient's oral cavity is obtained using an intraoral scanner according to an embodiment of the present disclosure.
FIG. 2A is a block diagram of an electronic apparatus and an intraoral scanner according to an embodiment of the present disclosure. FIG. 2B is a perspective view of an intraoral scanner according to an embodiment of the present disclosure.
FIG. 3 is a diagram illustrating a method for generating a three-dimensional image 320 of an oral cavity according to an embodiment of the present disclosure.
FIG. 4A and FIG. 4B are diagrams illustrating generating a three-dimensional image model in a first scanning step according to an embodiment of the present disclosure.
FIG. 5A and FIG. 5B are diagrams illustrating a screen when entering a second scanning step according to an embodiment of the present disclosure.
FIG. 6A and FIG. 6B are diagrams illustrating generating a three-dimensional image model in a second scanning step according to an embodiment of the present disclosure.
FIGS. 7A to 7C are diagrams illustrating comparison between scan data in a first scanning step and scan data in a second scanning step according to an embodiment of the present disclosure.
FIGS. 8A and 8B are diagrams illustrating generating a three-dimensional image model in a second scanning step according to an embodiment of the present disclosure.
FIGS. 9A to 9E are diagrams illustrating aligning a three-dimensional image model in a first scanning step and a three-dimensional image model in a second scanning step according to an embodiment of the present disclosure.
FIG. 10 is a flowchart illustrating a scan image processing method of a three-dimensional scanner according to an embodiment of the present disclosure.
FIG. 11 is a flowchart illustrating an operation of aligning a three-dimensional image model in a first scanning step and a three-dimensional image model in a second step according to an embodiment of the present disclosure.

### DETAILED DESCRIPTION

Embodiments of the present disclosure are illustrated for describing the technical spirit of the present disclosure. The scope of the claims according to the present disclosure is not limited to the embodiments described below or to the detailed descriptions of these embodiments.

All technical or scientific terms used herein have meanings that are generally understood by those skilled in the art to which the present disclosure pertains, unless otherwise specified. The terms used herein are selected for only more clear illustration of the present disclosure, and are not intended to limit the scope of claims in accordance with the present disclosure.

The expressions "include", "provided with", "have", and the like used herein should be understood as open-ended terms connoting the possibility of inclusion of other embodiments, unless otherwise mentioned in a phrase or sentence including the expressions.

A singular expression herein may include meanings of plurality, unless otherwise mentioned, and the same is applied to a singular expression stated in the claims. The terms "first", "second", etc. used herein are used to identify a plurality of components from one another, and are not intended to limit the order or importance of the relevant components.

The term "unit" used in the present disclosure means a software component or hardware component, such as a field-programmable gate array (FPGA) and an application specific integrated circuit (ASIC). However, a "unit" is not limited to software and hardware, it may be configured to be an addressable storage medium or may be configured to run on one or more processors. For example, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables. Functions provided in components and "unit" may be combined into a smaller number of components and "units" or further subdivided into additional components and "units."

The expression "based on" used herein is used to describe one or more factors that influences a decision, an action of judgment, or an operation described in a phrase or sentence including the relevant expression, and this expression does not exclude additional factor influencing the decision, the action of judgment, or the operation.

When a certain component is described as "coupled to" or "connected to" another component, this should be understood as having meaning that the certain component may be coupled or connected directly to the other component or that the certain component may be coupled or connected to the other component via a new intervening component.

In the present disclosure, the term "artificial intelligence (AI)" may refer to a technology for mimicking human learning, reasoning, or perceptual abilities and implementing the same as a computer, and may include concepts such as machine learning or symbolic logic. Machine learning (ML) may be an algorithm technology that classifies or learns the characteristics of input data on its own. The technology of artificial intelligence may analyze, using a machine learning algorithm, input data, learn the results of the analysis, and make judgments or predictions based on the results of the learning. In addition, technologies that use machine learning algorithms to mimic the cognitive and judgment functions of the human brain may also be understood as a category of artificial intelligence. For example, they may include technologies of linguistic understanding, visual understanding, inference/prediction, knowledge representation, and motion control.

In the present disclosure, machine learning may indicate the process of training a neural network model using the experience of processing data. It may indicate that computer software improves its own data processing capabilities through machine learning. A neural network model is constructed by modeling the correlations between data, which may be expressed by a plurality of parameters. The neural network model extracts and analyzes features from given data to derive correlations between the data, and this process is repeated to optimize the parameters of the neural network model, which is called machine learning. For example, the neural network model may learn the mapping (correlation) between input and output for data given as input-output pairs. Alternatively, even when only input data is given, the neural network model may learn the correlation by deriving the regularity between the given data.

In the present disclosure, an artificial intelligence learning model, a machine learning model, or a neural network model may be designed to implement the human brain structure on a computer and may include a plurality of network nodes that mimic neurons of the human nervous system and have weights. The plurality of network nodes may have interconnections with each other by mimicking the synaptic activity of neurons for transmitting and receiving signals through synapses. In an artificial intelligence learning model, a plurality of network nodes may be located at different depths of layers and may exchange data according to convolutional connections. The artificial intelligence learning model may be, for example, an artificial neural network model, a convolutional neural network model, or the like.

In the present disclosure, an electronic apparatus may use a data set A as input data and a data set B as output data according to a machine learning algorithm, to build a correlation model that models the correlation between the data set A and the data set B. The electronic apparatus may derive data b from data a using the built correlation model.

As used herein, the expressions "A, B, and C", "A, B, or C", "A, B, and/or C", "at least one of A, B, and C", or "at least one of A, B, or C," may indicate each of the listed items or all possible combinations of the listed items. For example, "at least one of A or B" may refer to (1) at least one A, (2) at least one B, or (3) at least one A and at least one B.

The expression "configured to" as used in the present disclosure may indicate, depending on the context, "set to", "having the ability to", "modified to", "made to", "capable of", or the like. This expression is not limited to the meaning of "specifically designed in hardware", and for example, a processor configured to perform a specific operation may indicate a generic purpose processor capable of performing the specific operation by executing software, or a special purpose computer that is structured through programming to perform the specific operation.

Hereinafter, embodiments of the present disclosure will be described with reference to the attached drawings. In the attached drawings, identical or corresponding components are assigned the same reference numerals. Additionally, in the following descriptions of embodiments, the redundant descriptions of identical or corresponding components may be omitted, but this does not imply that such components are excluded from the embodiments.

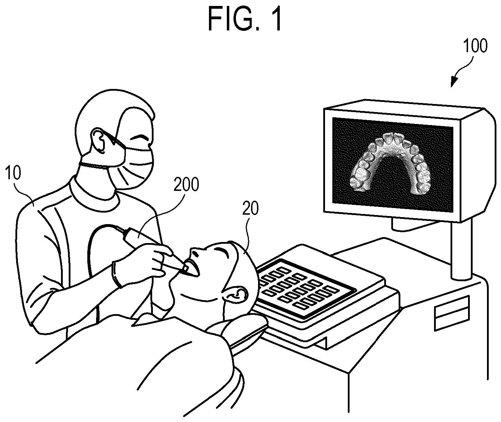

FIG. 1 is a diagram illustrating the state in which an image of a patient's oral cavity is obtained using a three-dimensional scanner 200 according to an embodiment of the present disclosure. According to an embodiment, the three-dimensional scanner 200 may be a dental medical device for obtaining an image of the oral cavity of an object 20. For example, the three-dimensional scanner 200 may be an intraoral scanner. As illustrated in FIG. 1, a user 10 (e.g., a dentist or a dental hygienist) may obtain an image of the oral cavity of the object 20 from the object 20 (e.g., a patient) using the three-dimensional scanner 200. As another example, the user 10 may obtain an image of the oral cavity of the object 20 from a diagnostic model (e.g., a plaster model or an impression model) that models the shape of the oral cavity of the object 20. Hereinafter, for the convenience of explanation, the description will be made based on scanning the oral cavity of the object 20 to obtain an image of the oral cavity of the object 20, but the present disclosure is not limited thereto, and it is also possible to obtain an image of another part (e.g., the ear of the object 20) of the object 20. The three-dimensional scanner 200 may have a form capable of being inserted into the oral cavity and retracted therefrom, and may be a handheld scanner in which the scan distance and the scan angle may be freely adjusted by the user 10.

The three-dimensional scanner 200 according to an embodiment may be inserted into the oral cavity of an object 20 and scan the inside of the oral cavity in a non-contact manner to obtain an image of the oral cavity. The image of the oral cavity may include at least one tooth, a gum, and an artificial structure that may be inserted into the oral cavity (e.g., an orthodontic device including a bracket and a wire, an implant, a denture, or an auxiliary orthodontic tool inserted into the oral cavity). The three-dimensional scanner 200 may radiate light onto the oral cavity of the object 20 (e.g., at least one tooth or a gum of the object 20) using a light source (or a projector) and receive light reflected from the oral cavity of the object 20 through a camera (or at least one image sensor).

A three-dimensional scanner 200 according to an embodiment may obtain a surface image of the oral cavity of the object 20 as a two-dimensional image, based on information received through the camera. The surface image of the oral cavity of the object 20 may include at least one tooth, a gum, or an artificial structure of the object 20, and a cheek, a tongue, or a lip of the object 20. The surface image of the oral cavity of the object 20 may be a two-dimensional image.

The two-dimensional image of the oral cavity obtained by the three-dimensional scanner 200 according to an embodiment may be transmitted to an electronic apparatus 100 connected through a wired or wireless communication network. The electronic apparatus 100 may be a computer device or a portable communication device. The electronic apparatus 100, based on the two-dimensional image of the oral cavity received from the three-dimensional scanner 200, may generate a three-dimensional image of the oral cavity (or a three-dimensional oral cavity image or a three-dimensional oral cavity model) that represents the oral cavity three-dimensionally. The electronic apparatus 100 may generate a three-dimensional image of the oral cavity by three-dimensionally modeling the internal structure of the oral cavity, based on the received two-dimensional image of the oral cavity.

The three-dimensional scanner 200 according to another embodiment may scan the oral cavity of the object 20 to obtain a two-dimensional image of the oral cavity, generate a three-dimensional image of the oral cavity, based on the obtained two-dimensional image of the oral cavity, and transmit the generated three-dimensional image of the oral cavity to the electronic apparatus 100.

The electronic apparatus 100 according to an embodiment may be connected to a cloud server (not shown). In the above case, the electronic apparatus 100 may transmit the two-dimensional image of the oral cavity of the object 20 or the three-dimensional image of the oral cavity to the cloud server, and the cloud server may store the two-dimensional image of the oral cavity of the object 20 or the three-dimensional image of the oral cavity received from the electronic apparatus 100.

According to another embodiment, in addition to a handheld scanner inserted into the oral cavity of the object 20 for use, a table scanner (not shown) fixed to a specific position may be used as the three-dimensional scanner. The table scanner may generate a three-dimensional image of an oral cavity diagnostic model by scanning the oral cavity diagnostic model. In the above case, since a light source (or projector) and a camera of the table scanner are fixed, the user may scan the oral cavity diagnostic model by moving an arm that fixes the oral cavity diagnostic model. Compared to the handheld scanner, the table scanner is less likely to cause noise by intervening with another object between the camera and the diagnostic model during scanning, and the embodiments of the present disclosure are applicable to the table scanner and other three-dimensional scanners, as well as the handheld scanner.

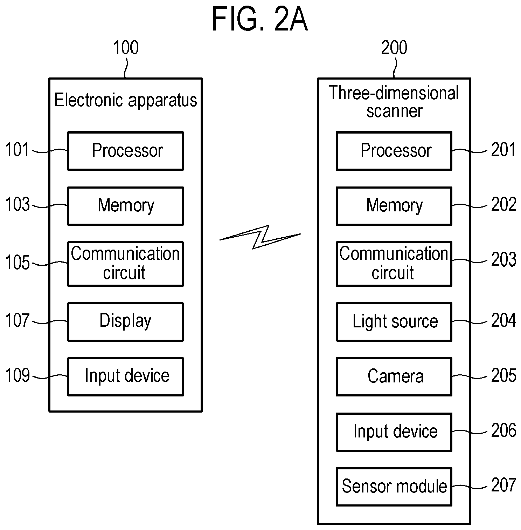

FIG. 2A is a block diagram of an electronic apparatus 100 and a three-dimensional scanner 200 according to an embodiment of the present disclosure. The electronic apparatus 100 and the three-dimensional scanner 200 may be connected to each other through a wired or wireless communication network, and may exchange various types of data with one another.

The three-dimensional scanner 200 according to an embodiment may include a processor 201, a memory 202, a communication circuit 203, a light source 204, a camera 205, an input device 206, and/or a sensor module 207. At least one of the components included in the three-dimensional scanner 200 may be omitted, or other components may be added to the three-dimensional scanner 200. Additionally or alternatively, some of the components may be implemented by integration, or may be implemented as a single or a plurality of entities. At least a part of the components in the three-dimensional scanner 200 may be connected to each other through a bus, a general purpose input/output (GPIO), a serial peripheral interface (SPI), or a mobile industry processor interface (MIPI) to exchange data and/or signals with one another.

The processor 201 of the three-dimensional scanner 200 according to an embodiment is a configuration capable of performing operations or data processing related to control and/or communication of the respective components of the three-dimensional scanner 200, and may be operatively connected to the components of the three-dimensional scanner 200. The processor 201 may load commands or data received from other components of the three-dimensional scanner 200 to the memory 202, process the commands or data stored in the memory 202, and store the resultant data. The memory 202 of the three-dimensional scanner 200 according to an embodiment may store instructions for the operations of the processor 201 described above.

According to an embodiment, the communication circuit 203 of the three-dimensional scanner 200 may establish a wired or wireless communication channel with an external device (e.g., the electronic apparatus 100), and transmit and receive various data to and from the external device. According to an embodiment, the communication circuit 203 may include at least one port to be connected to the external device with a wired cable in order to communicate with the external device via a wire. In the above case, the communication circuit 203 may perform communication with the external device connected via a wired connection through at least one port. According to an embodiment, the communication circuit 203 may include a cellular communication module and may be configured to be connected to a cellular network (e.g., 3G, LTE, 5G, Wibro, or Wimax). According to an embodiment, the communication circuit 203 may include a short-range communication module to transmit and receive data to and from the external device using short-range communication (e.g., Wi-Fi, Bluetooth, Bluetooth Low Energy (BLE), or UWB), but is not limited thereto. According to an embodiment, the communication circuit 203 may include a non-contact communication module for non-contact communication. The non-contact communication may include at least one non-contact type proximity communication technology, such as near field communication (NFC), radio frequency identification (RFID) communication, or magnetic secure transmission (MST) communication.

The light source 204 of the three-dimensional scanner 200 according to an embodiment may radiate light toward the oral cavity of the object 20. For example, the light radiated from the light source 204 may be structured light having a predetermined pattern (e.g., a stripe pattern in which straight lines of different colors appear continuously). The pattern of the structured light may be generated using, for example, a pattern mask or a DMD (digital micro-mirror device), but it is not limited thereto. The camera 205 of the three-dimensional scanner 200 according to an embodiment may receive the light reflected from the oral cavity of the object 20 to obtain images of the oral cavity of the object 20. The camera 205 may be configured as, for example, one camera. Alternatively, the camera 205 may include a left camera corresponding to the left eye view and a right camera corresponding to the right eye view to produce a three-dimensional image according to optical triangulation. In addition, the camera 205 may include at least one image sensor, such as a CCD sensor or a CMOS sensor.

The input device 206 of the three-dimensional scanner 200 according to an embodiment may receive a user input for controlling the three-dimensional scanner 200. The input device 206 may include a button for receiving a push operation of the user 10, a touch panel for detecting a touch of the user 10, and a voice recognition device including a microphone. For example, the user 10 may control the start or stop of scanning using the input device 206.

The sensor module 207 of the three-dimensional scanner 200 according to an embodiment may detect the operating state of the three-dimensional scanner 200 or the external environmental state (e.g., the user's action) and generate an electrical signal corresponding to the detected state. The sensor module 207 may include at least one of a gyro sensor, an acceleration sensor, a gesture sensor, a proximity sensor, or an infrared sensor. Here, the user 10 may control the start or stop of scanning using the sensor module 207. For example, when the user 10 moves while holding the three-dimensional scanner 200, the three-dimensional scanner 200 may control the processor 201 to start a scanning operation when the angular velocity measured by the sensor module 207 exceeds a setting value.

According to an embodiment, the three-dimensional scanner 200 may receive a user input to start scanning through the input device 206 of the three-dimensional scanner 200 or the input device 206 of the electronic apparatus 100, or start scanning according to processing by the processor 201 of the three-dimensional scanner 200 or the processor 201 of the electronic apparatus 100. When the user 10 scans the inside of the oral cavity of the object 20 using the three-dimensional scanner 200, the three-dimensional scanner 200 may generate two-dimensional images of the oral cavity of the object 20 and transmit the two-dimensional images of the oral cavity of the object 20 to the electronic apparatus 100 in real-time. The electronic apparatus 100 may display the received two-dimensional images of the oral cavity of the object 20 through the display. In addition, the electronic apparatus 100 may generate (construct) a three-dimensional image of the oral cavity of the object 20 based on the two-dimensional images of the oral cavity of the object 20 and display the three-dimensional image of the oral cavity through the display. The electronic apparatus 100 may display the three-dimensional image being generated in real-time on the display.

The electronic apparatus 100 according to an embodiment may include one or more processors 101, one or more memories 103, a communication circuit 105, a display 107, and/or an input device 109. At least one of the components included in the electronic apparatus 100 may be omitted, or other components may be added to the electronic apparatus 100. Additionally or alternatively, some of the components may be implemented by integration, or may be implemented as a single or a plurality of entities. At least a part components in the electronic apparatus 100 may be connected to each other via a bus, a general purpose input/output (GPIO), a serial peripheral interface (SPI), or a mobile industry processor interface (MIPI), and may exchange data and/or signals with each other.

According to an embodiment, one or more processors 101 of the electronic apparatus 100 may be configured to perform operations or data processing related to control and/or communication of the respective components (e.g., the memory 103) of the electronic apparatus 100. For example, one or more processors 101 may be operatively connected to the components of the electronic apparatus 100. One or more processors 101 may load commands or data received from other components of the electronic apparatus 100 to one or more memories 103, process commands or data stored in one or more memories 103, and store resultant data.

According to an embodiment, one or more memories 103 of the electronic apparatus 100 may store instructions for the operation of one or more processors 101. One or more memories 103 may store data (e.g., two-dimensional images of the oral cavity obtained through oral cavity scanning) received from the three-dimensional scanner 200.

According to an embodiment, the communication circuit 105 of the electronic apparatus 100 may establish a wired or wireless communication channel with an external device (e.g., the three-dimensional scanner 200 or a cloud server) and transmit and receive various data to and from the external device. According to an embodiment, the communication circuit 105 may include at least one port for connection with the external device using a wired cable in order to communicate with the external device through wires. In the above case, the communication circuit 105 may perform communication with the external device connected via a wired connection through at least one port. According to an embodiment, the communication circuit 105 may include a cellular communication module and may be configured to be connected to a cellular network (e.g., 3G, LTE, 5G, Wibro, or Wimax). According to an embodiment, the communication circuit 105 may include a short-range communication module to transmit and receive data to and from the external device using short-range communication (e.g., Wi-Fi, Bluetooth, Bluetooth Low Energy (BLE), or UWB), but is not limited thereto. According to an embodiment, the communication circuit 105 may include a non-contact communication module for non-contact communication. The non-contact communication may include at least one non-contact type proximity communication technology, such as near field communication (NFC), radio frequency identification (RFID) communication, or magnetic secure transmission (MST) communication.

The display 107 of the electronic apparatus 100 according to an embodiment may display various screens under the control of the processor 101. The processor 101 may display the two-dimensional image of the oral cavity of the object 20 received from the three-dimensional scanner 200 and/or the three-dimensional image of the oral cavity in which the internal structure of the oral cavity is modeled in three dimensions through the display 107. For example, the two-dimensional image and/or three-dimensional image of the oral cavity may be displayed using a specific application program. In the above case, the user 10 may edit, save, or delete the two-dimensional image and/or three-dimensional image of the oral cavity.

The input device 109 of the electronic apparatus 100 according to an embodiment may receive commands or data to be used by the components (e.g., one or more processors 101) of the electronic apparatus 100 from the outside (e.g., the user) of the electronic apparatus 100. The input device 109 may include, for example, a microphone, mouse, or keyboard. According to an embodiment, the input device 109 may be implemented as a touch sensor panel that is connected to the display 107 and is able to recognize the contact or proximity of various external objects.

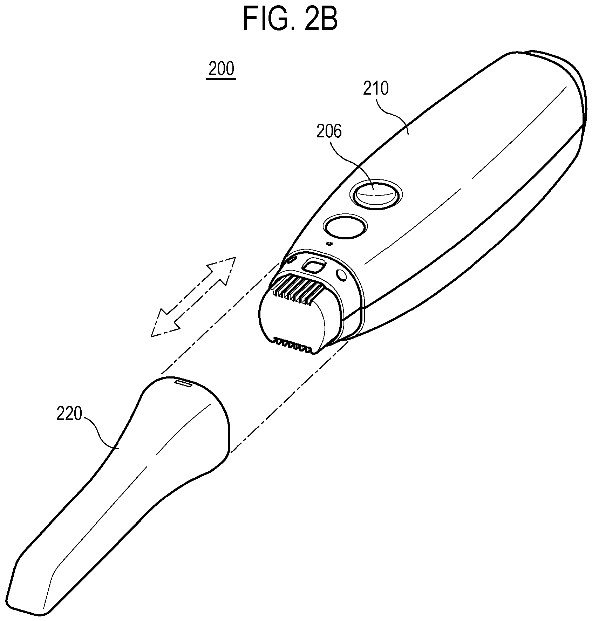

FIG. 2B is a perspective view of a three-dimensional scanner 200 according to an embodiment of the present disclosure. The three-dimensional scanner 200 according to an embodiment may include a main body 210 and a probe tip 220. The main body 210 of the three-dimensional scanner 200 may be shaped to be easily gripped and used by hands of the user 10. The probe tip 220 may be shaped for easy insertion into and removal from the oral cavity of the object 20. In addition, the main body 210 may be coupled to and detached from the probe tip 220. The components of the three-dimensional scanner 200 described in FIG. 2A may be disposed inside the main body 210. The main body 210 may have an opening at one end through which light output from the light source 204 may be emitted to the object 20. The light emitted through the opening may be reflected by the object 20 and may enter back through the opening. Here, the reflected light entering through the opening may be captured by the camera to generate images of the object 20. The user 10 may start scanning using the input device 206 (e.g., a button) of the three-dimensional scanner 200. For example, when the user 10 touches or presses the input device 206, light from the light source 204 may be emitted toward the object 20.

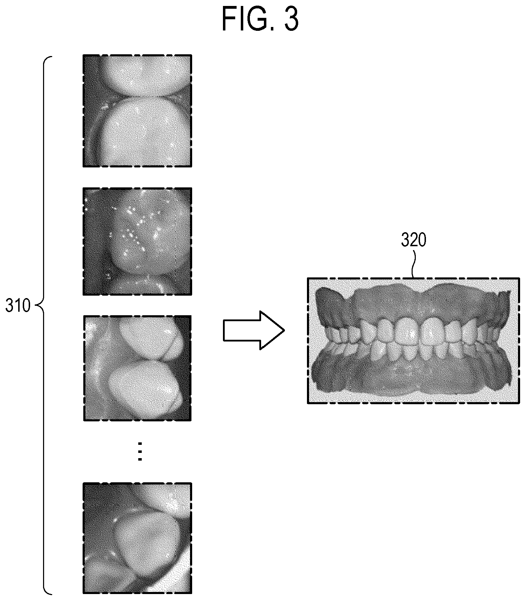

FIG. 3 is a diagram illustrating a method for generating a three-dimensional image 320 of an oral cavity according to an embodiment of the present disclosure. The user 10 may scan the inside of the oral cavity of the object 20 while moving the three-dimensional scanner 200, and in this case, the three-dimensional scanner 200 may obtain a plurality of two-dimensional images 310 of the oral cavity of the object 20. For example, the three-dimensional scanner 200 may obtain a two-dimensional image of a region including the front teeth of the object 20, a two-dimensional image of a region including the molars of the object 20, and the like. The three-dimensional scanner 200 may transmit the plurality of obtained two-dimensional images 310 to the electronic apparatus 100.

The electronic apparatus 100 according to an embodiment may convert each of the two-dimensional images 310 for the oral cavity of the object 20 into a set of multiple points having three-dimensional coordinate values. For example, the electronic apparatus 100 may convert the respective two-dimensional images 310 into a point cloud, which is a set of data points having three-dimensional coordinate values. A point cloud set, which is a three-dimensional coordinate values based on the plurality of two-dimensional images 310, may be stored as raw data on the oral cavity of the object 20. The electronic apparatus 100 may complete the entire tooth model by aligning the point cloud, which is a set of data points having three-dimensional coordinate values.

The electronic apparatus 100 according to an embodiment may reconfigure (rebuild) a three-dimensional image of the oral cavity. For example, the electronic apparatus 100 may reconfigure a plurality of points by merging point cloud sets stored as raw data using a Poisson algorithm and convert them into a closed three-dimensional surface, thereby reconfiguring the three-dimensional image 320 for the oral cavity of the object 20.

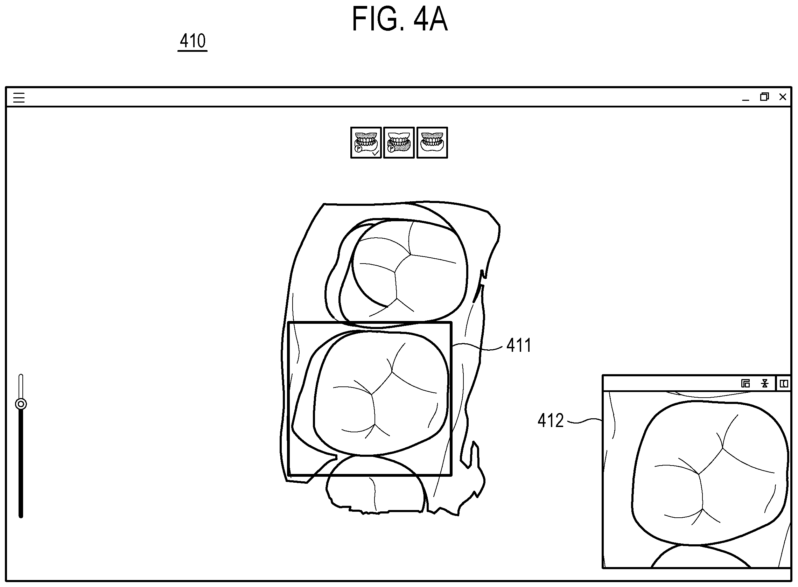

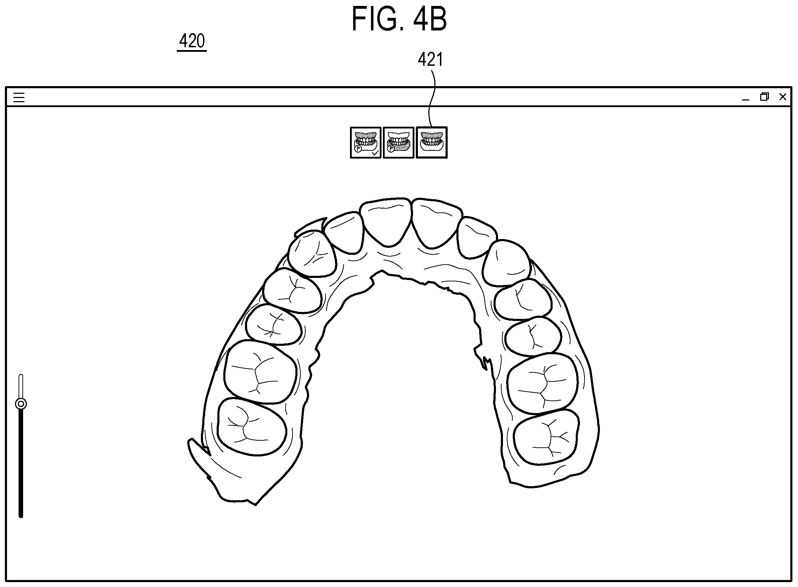

FIG. 4A and FIG. 4B are diagrams illustrating generating a three-dimensional image model in a first scanning step according to an embodiment of the present disclosure.

The electronic apparatus 100 may obtain first scan data values for the surface of the object 20 by first scanning by the three-dimensional scanner 200. Here, the obtained first scan data values may include three-dimensional coordinate values. In addition, the first scanning may be one image-capturing by the camera of the three-dimensional scanner, or may correspond to any number of image-capturings required to generate the three-dimensional image model. In an embodiment, the first scanning may correspond to scanning for a predetermined period of time. The three-dimensional coordinate values may be generated based on two-dimensional image data obtained by the scanner. In an embodiment, the first scan data values may include three-dimensional volume data in the form of voxels. In addition, the electronic apparatus 100 may generate a first three-dimensional image model based on the first scan data values. Here, the first three-dimensional image model may indicate the three-dimensional image model in the first scanning step. The first scan data values for the surface of the object are obtained by the first scanning.

For example, during the oral cavity treatment process for a patient, oral cavity scanning in the initial state before the start of treatment, oral cavity scanning in the state where the patient's teeth are partially cut and preparation for treatment is completed before full-scale treatment (e.g., prosthetic treatment), and oral cavity scanning in the state where a prosthesis is inserted into the cut teeth may be required, respectively. The oral cavity scanning in the initial state before the start of treatment may be referred to as "pre-op" scanning, the oral cavity scanning in the state where the patient's teeth are partially cut and preparation for treatment is completed may be referred to as "prep" scanning, and the oral cavity scanning in the state where full-scale treatment is completed may be referred to as "scanning after prosthesis insertion". In this example, the pre-op scanning preceding the prep scanning may be the first scanning, and the prep scanning preceding the scanning after prosthesis insertion may be the first scanning.

As in the case of the prep scanning, in the process of generating the first three-dimensional image model, there may not be a three-dimensional image model generated in advance. In this case, the entire oral cavity of the patient to be scanned must be scanned in the first scanning.

As in the case of the pre-op scanning, if there is a three-dimensional image model generated in advance, only a part of the oral cavity of the patient to be scanned may be scanned in the first scanning.

Referring to FIG. 4A, the electronic apparatus 100 may display, through the display 107, a screen 410 including a three-dimensional image of the oral cavity of the object 20, which is being generated in the first scanning step, in real time. The electronic apparatus 100 may display, on the display 107, an area 411 currently being scanned by the three-dimensional scanner 200 in the three-dimensional image of the oral cavity of the object 20, which is being generated. For example, the electronic apparatus 100 may display an area 411 currently being scanned by the three-dimensional scanner 200 through a box-shaped marking, thereby allowing the user to recognize which part is currently being scanned. In addition, the electronic apparatus 100 may display an area 412 including a two-dimensional image currently being obtained by the three-dimensional scanner 200 on the display 107.

Referring to FIG. 4B, the electronic apparatus 100 may display, on the display 107, a screen 420 including a first three-dimensional image model generated after the first scanning is completed. The generated first three-dimensional image model may be stored in the memory 103. In addition, the electronic apparatus 100 may display an icon 421 indicating second scanning on the display 107. The electronic apparatus 100 may perform a second scanning step when an input to the icon 421 indicating second scanning is obtained. However, in addition to selecting the icon 421 indicating the second scanning on the screen 420 including the first three-dimensional image model as a result of the first scanning, the second scanning step may also be executed in other ways, such as when the first scanning is stopped, when a specific time elapses after the start of the first scanning, or when a separate prompt is output to obtain an input from the user.

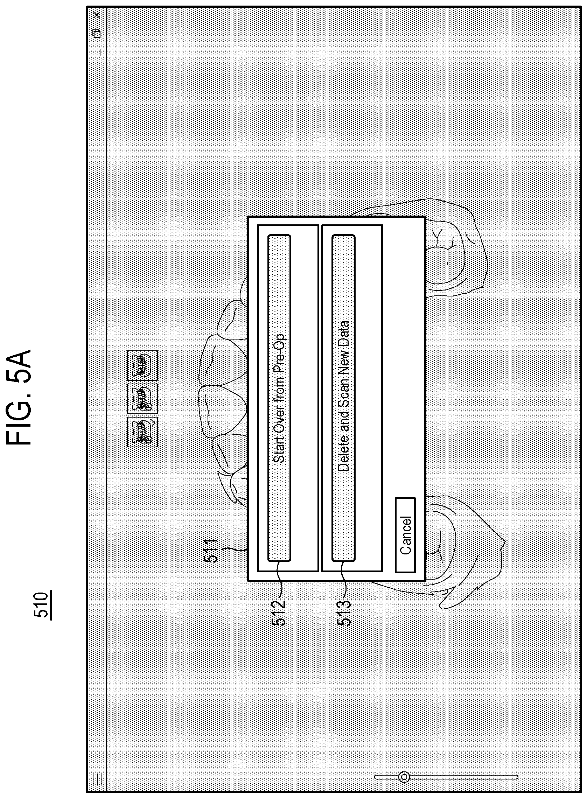

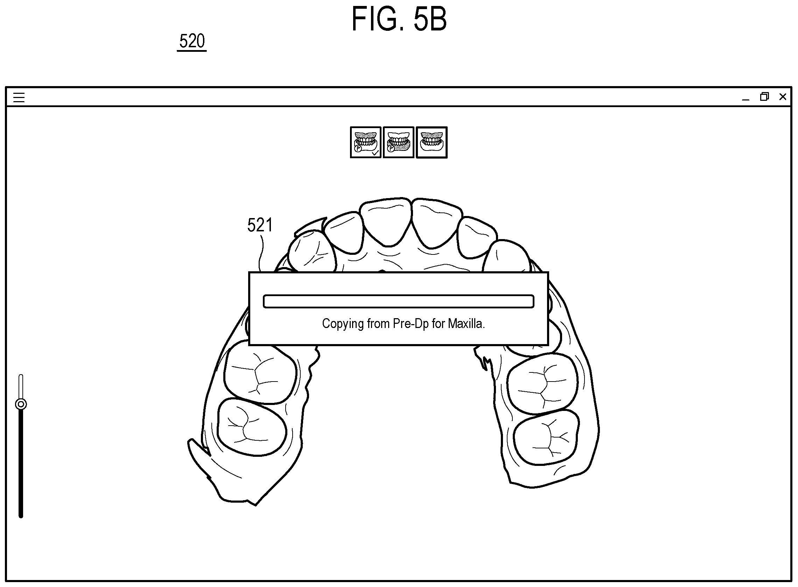

FIG. 5A and FIG. 5B are diagrams illustrating a screen when entering a second scanning step according to an embodiment of the present disclosure. In the above-mentioned example, if the first scanning is pre-op scanning, the second scanning may be prep scanning, and if the first scanning is prep scanning, the second scanning may be scanning after prosthesis insertion.

Referring to FIG. 5A, if the second scanning step is initiated, the electronic apparatus 100 may display a screen 510 including an area 511 in which a user's selection for the second scanning step is input on the display 107. In an embodiment, the area 511 for receiving a user's selection may include a button 512 for instructing to generate a second three-dimensional image model, based on the first three-dimensional image model generated in the first scanning step, and a button 513 for instructing to generate a second three-dimensional image model through scan data newly obtained in a second scanning step, regardless of the first three-dimensional image model generated in the first scanning step (i.e., not based on data included in the first three-dimensional image model). The button 512 may include text indicating generating a second three-dimensional image model based on the first three-dimensional image model generated in the first scanning step. Additionally, the button 513 may include text indicating generating a second three-dimensional image model through scan data newly obtained in the second scanning step, regardless of the first three-dimensional image model generated in the first scanning step. The electronic apparatus 100 may receive an input to the button 512 or the button 513 from the user through the input device 109. In an embodiment, when entering the second scanning step, the electronic apparatus 100 may generate a second three-dimensional image model by a predetermined default method (for example, a method of generating a second three-dimensional image model based on the first three-dimensional image model generated in the first scanning step or a method of generating a second three-dimensional image model through scan data newly obtained in the second scanning step independently of the first three-dimensional image model generated in the first scanning step), instead of an input received from the user. In addition, in an embodiment, the electronic apparatus 100 may receive, from the user, an input regarding whether to output the area 511 in which a user's selection is input or to generate a second three-dimensional image model according to a predetermined default method in advance when entering the second scanning step. In addition, any combination of the above-described methods may all be included in the embodiment. The electronic apparatus 100 may output the area 511 for receiving an input of user's selection for a method of generating a second three-dimensional image model or generate a second three-dimensional image model according to a predetermined default method when entering the second scanning step.

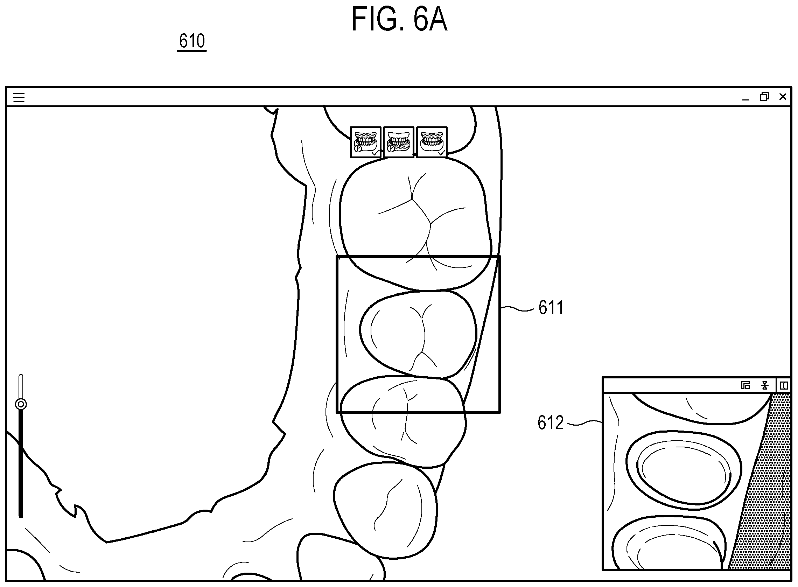

In an embodiment, if an input to the button 512 is obtained, the electronic apparatus 100 may generate a second three-dimensional image model in the second scanning step by automatically replacing at least a part of the first scan data values with the second scan data values . Here, "automatically" means that the electronic apparatus 100 may replace at least a part of the first scan data values with the second scan data values without the user's intervention. As described above, if the electronic apparatus 100 automatically replaces at least a part of the first scan data values with the second scan data values, the user does not need to specify the first scan data values to be replaced with the second scan data values. In an embodiment, the electronic apparatus 100 may obtain second scan data values for the surface of the object 20 by second scanning by the three-dimensional scanner performed after generation of the first three-dimensional image model. Here, the second scan data values may include three-dimensional coordinate values. In addition, the second scanning may be one image-capturing by the camera of the three-dimensional scanner, or may correspond to any number of image-capturings required to generate the three-dimensional image model. In an embodiment, the second scanning may correspond to scanning for a predetermined period of time. The three-dimensional coordinate values may be generated based on two-dimensional image data obtained by the scanner. In an embodiment, the second scan data values may include three-dimensional volume data in the form of voxels. The electronic apparatus 100 may compare the first scan data values and the second scan data values to determine first sub-data values, which are at least a part of the first scan data values, and second sub-data values, which are at least a part of the second scan data values. The electronic apparatus 100 may generate the second three-dimensional image model based on third scan data values obtained by replacing the first sub-data values in the first scan data values with the second sub-data values. The determination of the first sub-data values and the second sub-data values by the electronic apparatus 100 will be specifically described with reference to FIGS. 6A to 7B below.

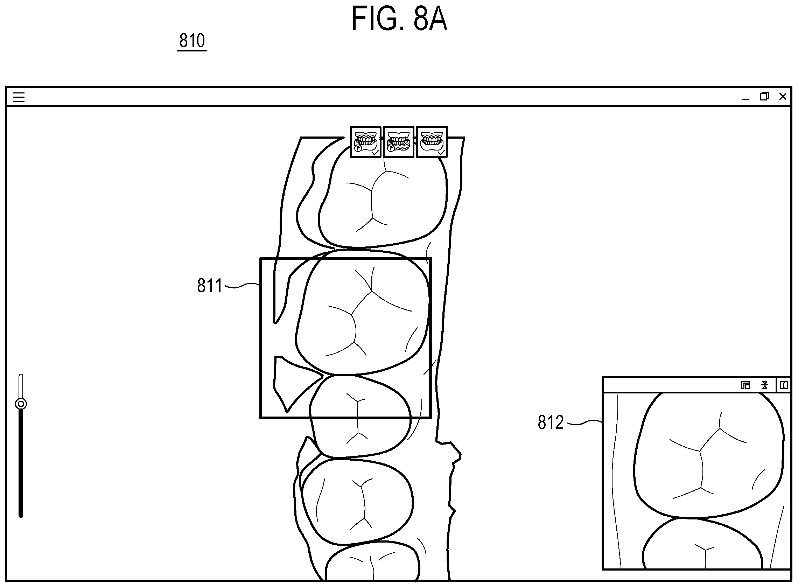

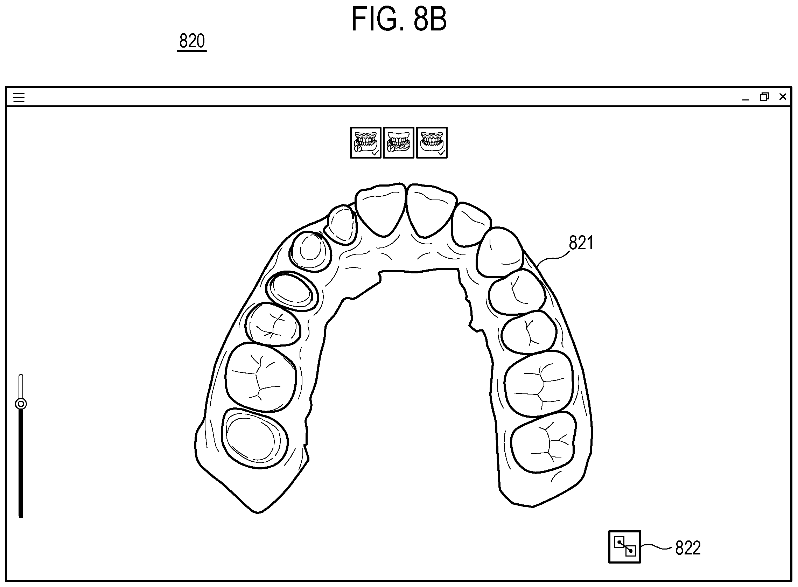

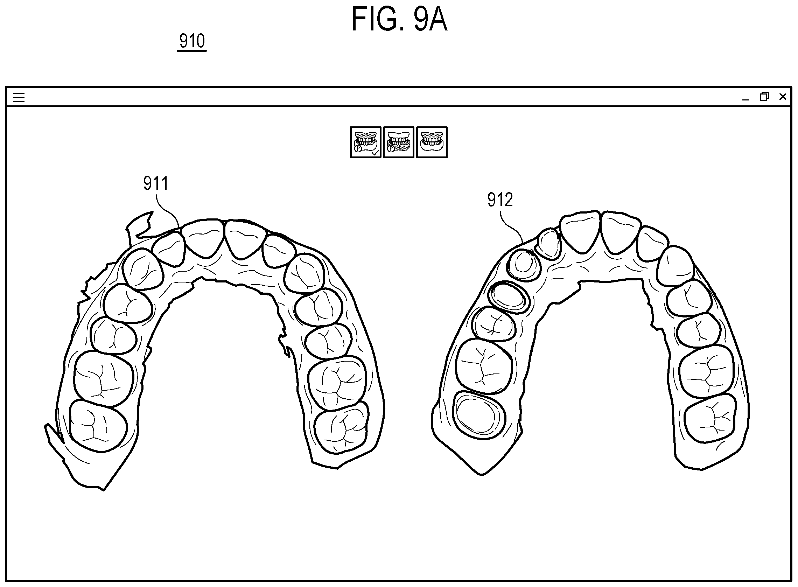

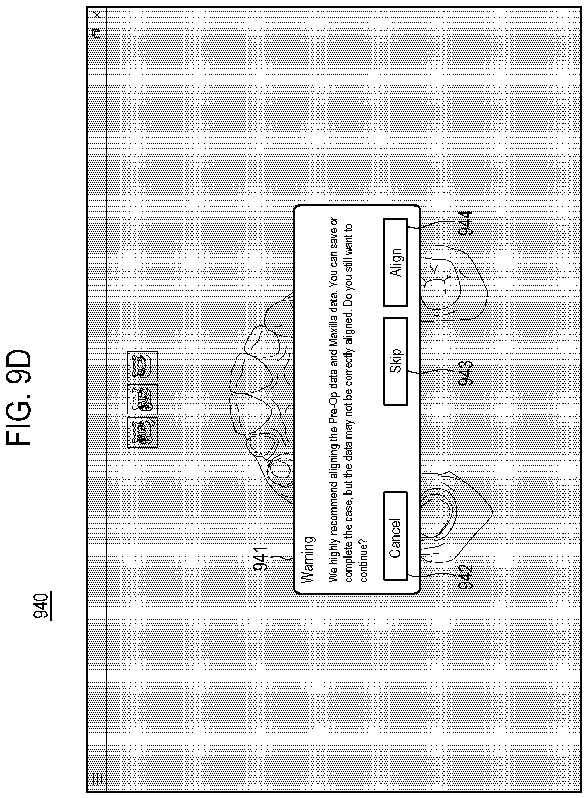

In an embodiment, if an input to the button 513 is obtained, the electronic apparatus 100 may generate the second three-dimensional image model through scan data newly obtained in the second scanning step independently of the first three-dimensional image model generated in the first scanning step. For example, in response to an input to the button 513, the electronic apparatus 100 may generate the second three-dimensional image model based on the second scan data, instead of replacing the first sub-data values in the first scan data values with the second sub-data values. In other words, the electronic apparatus 100 may generate the second three-dimensional image model based on the second scan data values obtained in the second scanning in the same or similar manner as the process of generating the first three-dimensional image model based on the first scan data values obtained in the first scanning. In this case, second scanning is required for the entire surface of the object 20. The generation of the second three-dimensional image model through the scan data newly obtained in the second scanning step without using the first three-dimensional image model will be described in detail with reference to FIGS. 8A and 8B below. In addition, in this case, in order to compare the first three-dimensional image model and the second three-dimensional image model, a process of aligning the first three-dimensional image model and the second three-dimensional image model may be further required. The process of aligning the first three-dimensional image model and the second three-dimensional image model will be specifically described with reference to FIGS. 9A to 9E below.

Referring to FIG. 5B, the electronic apparatus 100 may replicate and utilize the first three-dimensional image model as a basic model for the process of generating the second three-dimensional image model (e.g., when an input to the button 512 is obtained in FIG. 5A). That is, the electronic apparatus 100 may generate the second three-dimensional image model from the first three-dimensional image model by making changes based on newly scanned data for some areas in the second scanning step to be described below. The electronic apparatus 100 may replicate the first three-dimensional image model and output a screen 520 including an area 521 indicating a replication progress of the first three-dimensional image model on the display 107 while the replication is in progress. Here, according to the embodiment, the first three-dimensional image model may be replicated at various times. For example, the replication of the first three-dimensional image model may be started or completed in advance before or during the determination of a method used to generate the second three-dimensional image model from among the methods of generating the second three-dimensional image model described in FIG. 5A (i.e., before the input to the button 512 or button 513 is obtained in FIG. 5A). In this case, if a method of generating the second three-dimensional image model, based on the first three-dimensional image model generated in the first scanning step, is determined, it is possible to quickly proceed to the second scanning step. If a method of generating the second three-dimensional image model through scan data newly obtained in the second scanning step, independently of the first three-dimensional image model generated in the first scanning step, is determined, the duplication of the first three-dimensional image model may be stopped and the duplicated model may be deleted. Alternatively, after a method of generating the second three-dimensional image model is determined from among the methods of generating the second three-dimensional image model, only if a method of generating the second three-dimensional image model based on the first three-dimensional image model generated in the first scanning step is determined, the first three-dimensional image model may be replicated.

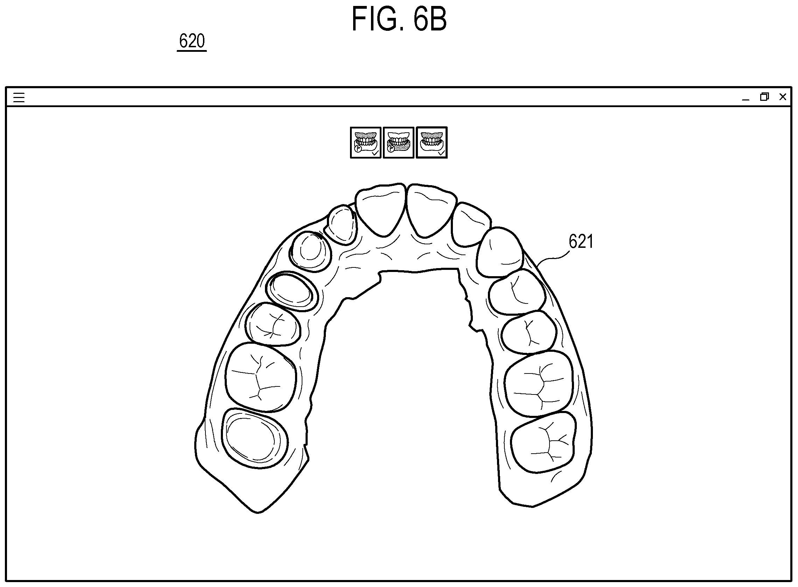

FIG. 6A and FIG. 6B are diagrams illustrating generating a three-dimensional image model in a second scanning step according to an embodiment of the present disclosure.

In the process of generating the second three-dimensional image model in the second scanning step, a first three-dimensional image model that was previously generated may exist. Therefore, in the second scanning, only a part of the oral cavity of the patient to be scanned, which has changed after the first scanning (e.g., a tooth that has been cut or a part where a prosthesis has been inserted), may be scanned. In addition, the electronic apparatus 100 may compare the scanned part with data on the first three-dimensional image model and change only the part that has changed based on the data of the second scanning. In an embodiment, the second scanning may be performed in such a manner that it starts from an area that has not changed after the first scanning (an area where the scan data values based on the first scanning will remain) and continues to an area that has changed after the first scanning (an area where the scan data values based on the first scanning will be replaced with the scan data values based on the second scanning).

Referring to FIG. 6A, the electronic apparatus 100 may display, through the display 107, a screen 610 including a three-dimensional image of the oral cavity of the object 20, which is being generated in the second scanning step, in real time. The electronic apparatus 100 may display, on the display 107, an area 611 currently being scanned by the three-dimensional scanner 200 in the three-dimensional image of the oral cavity of the object 20, which is being generated. For example, the electronic apparatus 100 may display an area 611 currently being scanned by the three-dimensional scanner 200 using a box-shaped marking, and thus the user may recognize which part is currently being scanned. In addition, the electronic apparatus 100 may display, on the display 107, an area 612 including a two-dimensional image currently being obtained through the three-dimensional scanner 200. For example, a first three-dimensional image model (e.g., an oral cavity image with teeth not cut at the time of the pre-op scanning) may be displayed in the area 611, and a two-dimensional image scanned in the second scanning (e.g., an oral cavity image with teeth cut at the time of the prep scanning) may be displayed in the area 612.

Referring to FIG. 6B, the electronic apparatus 100 may display, on the display 107, a screen 620 including a second three-dimensional image model 621 generated after the second scanning is completed. The generated second three-dimensional image model 621 may be stored in the memory 103.

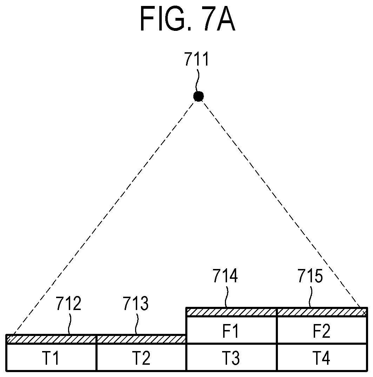

FIGS. 7A to 7C are diagrams illustrating comparison between scan data in a first scanning step and scan data in a second scanning step according to an embodiment of the present disclosure.

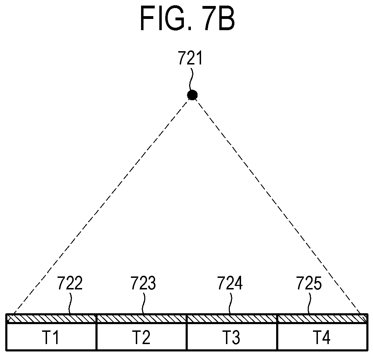

In FIGS. 7A to 7C, T1 to T4 may represent, for example, teeth. In addition, F1 and F2 may represent a part of teeth that exists in the first scanning step but is cut away in the second scanning step. In the first scanning, the surfaces of T1, T2, F1, and F2 (the portion indicated by ▨ in FIG. 7A, i.e., 712, 713, 714, and 715) may be scanned. In the second scanning, the surfaces of T1 to T4 (the portion indicated by 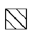 in FIG. 7B, i.e., 722, 723, 724, and 725) may be scanned. For convenience of explanation, it is assumed that the areas scanned in the first scanning and the second scanning are the same.

The electronic apparatus 100 may determine a virtual focus 711 of the three-dimensional scanner in the first scanning. In an embodiment, the virtual focus 711 may be, for example, the position determined according to a position of the light source 204 of the three-dimensional scanner or positions of teeth, a position of the camera 205 or positions of teeth, or the geometrical relationship of these configurations. Alternatively, the virtual focus 711 may be determined as a reference point that is specified as one according to an algorithm so as to correspond to the first three-dimensional image model.

In addition, the electronic apparatus 100 may determine a virtual focus 721 of the three-dimensional scanner in the second scanning. In an embodiment, the virtual focus 721, like the virtual focus 711 in the first scanning, may be, for example, the position determined according to a position of the light source 204 of the three-dimensional scanner or positions of teeth, a position of the camera 205 or positions of teeth, or the geometrical relationship of these configurations. Alternatively, the virtual focus 721 may be determined as a specific point at a position corresponding to the virtual focus 711 in the first scanning.

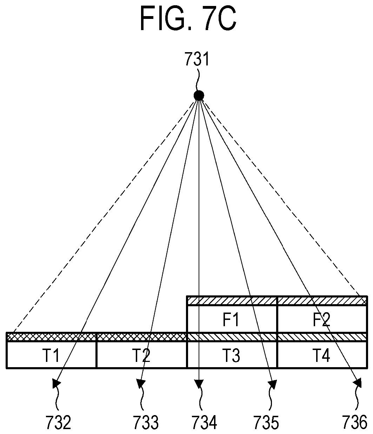

In an embodiment, the electronic apparatus 100 may position the surfaces of T1, T2, F1, and F2 scanned by the first scanning (the portion indicated by ▨ in FIG. 7A) and the surfaces of T1 to T4 scanned by the second scanning (the portion indicated by 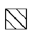 in FIG. 7B) in one virtual space. As a result, a virtual focus 731, the surfaces of T1, T2, F1, and F2 (the portion indicated by ▨ in FIG. 7C) (hereinafter, a first surface), and the surfaces of T1 to T4 (the portion indicated by 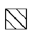 in FIG. 7C) (hereinafter, a second surface) may be positioned in the virtual space. The electronic apparatus 100 may determine first vectors 732, 733, 734, 735, and 736 connecting the virtual focus 711 of the three-dimensional scanner and first scan data values (the surfaces of T1, T2, F1, and F2 indicated by ▨ in FIG. 7C). In an embodiment, the first vectors 732, 733, 734, 735, and 736 may be vectors extending from the virtual focus 731 through respective voxel values corresponding to the first scan data values in the virtual space. Although, for convenience, only five vectors 732, 733, 734, 735, and 736 are illustrated in FIG. 7C, the vectors may be generated for all the first scan data values in the virtual space.

The electronic apparatus 100 determines whether or not the determined vectors 732, 733, 734, 735, and 736 intersect second scan data values in the virtual space. In an embodiment, determining whether or not the vectors intersect the second scan data values includes determining whether or not the vectors intersect the second surface. In another embodiment, determining whether or not the vectors intersect the second scan data values includes determining whether a distance between each of the vectors and a second scan data value that is at a closest vertical distance is less than or equal to a predetermined threshold. The electronic apparatus 100 may determine, if at least one of the vectors intersects at least one of the second scan data values, the first scan data value associated with the vector intersecting the second scan data value as a first sub-data values. In this case, the electronic apparatus 100 may determine the second scan data value intersecting as a second sub-data values.

In the embodiment illustrated in FIG. 7C, the vectors 732, 733, 734, 735, and 736 intersect the second surface (indicated by 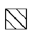 in FIG. 7C) comprised of second scan data values in the virtual space. The first scan data values associated with the vectors 732, 733, 734, 735, and 736 intersecting the second scan data values or the surface configured as the second scan data values, as described above, may be determined as first sub-data values. In this case, the second scan data values intersecting the vectors 732, 733, 734, 735, and 736 may be determined as second sub-data values. As a result, the scan data values of the first surface associated with the vectors 732, 733, 734, 735, and 736 may be replaced with the scan data values of the second surface intersecting the vectors 732, 733, 734, 735, and 736. Then, based on the result of replacing the scan data values of the first surface with the scan data values of the second surface intersecting the vectors 732, 733, 734, 735, and 736, a second three-dimensional image model may be generated.

In another embodiment, the electronic apparatus 100 may determine, if the second scan data value intersecting the vector is located within a predetermined distance from the first scan data value associated with the vector, that the relevant vector does not intersect the second scan data value. In other words, only if the second scan data value intersecting the vector and the first scan data value associated with the vector are further than a predetermined distance, the vector may be determined to intersect the second scan data value. The predetermined distance may be a distance on a predetermined three-dimensional coordinate. For example, in FIG. 7C, it can be seen that the second scan data value intersecting the vectors 732 and 733 and the first scan data value associated with the vector are the same or very close. That is, the distance between the second scan data value on the second surface intersecting the vectors 732 and 733 and the first scan data value associated with the vectors 732 and 733 may be within a predetermined distance. In this case, the vectors 732 and 733 may not be determined to intersect the second scan data value or the second surface.

On the other hand, the distance between the second scan data value on the second surface intersecting the vectors 734, 735, and 736 and the first scan data value associated with the vectors 734, 735, and 736 may be greater than a predetermined distance. In this case, the vectors 734, 735 and 736 may be determined to intersect the second scan data value or the second surface.

Although, in the embodiment in FIG. 7C, the vectors 732, 733, 734, 735, and 736 connecting the virtual focus 731 and the first scan data values are determined in the virtual space and then it is determined whether or not the vectors 732, 733, 734, 735, and 736 intersect the second scan data values, thereby deleting data values corresponding to noise from the first scan data values, the present disclosure is not limited thereto. In another embodiment of the present disclosure, vectors connecting the virtual focus and the second scan data values are determined in a virtual space, and then whether the vectors intersect the first scan data values are determined, thereby determining the first sub-data values and the second sub-data values .

FIGS. 8A and 8B are diagrams illustrating generating a three-dimensional image model in a second scanning step according to an embodiment of the present disclosure.

In the process of generating a second three-dimensional image model in the prep scanning step, the first three-dimensional image model, which was previously generated, may exist, but if the user wishes to generate a second three-dimensional image model based only on new second scanning, independently of the first three-dimensional image model, the electronic apparatus 100 may not utilize the first three-dimensional image model in the process of generating the second three-dimensional image model. In this case, the entire oral cavity of the patient to be scanned must be scanned in the second scanning.

Referring to FIG. 8A, the electronic apparatus 100 may display, through the display 107, a screen 810 including a three-dimensional image of the oral cavity of the object 20, which is being generated in the prep scanning step, in real time. The electronic apparatus 100 may display, on the display 107, an area 811 currently being scanned by the three-dimensional scanner 200 in the three-dimensional image of the oral cavity of the object 20, which is being generated. For example, the electronic apparatus 100 may display the area 811 currently being scanned by the three-dimensional scanner 200 using a box-shaped marking, through which the user may recognize which part is currently being scanned. In addition, the electronic apparatus 100 may display an area 812 including a two-dimensional image currently being obtained through the three-dimensional scanner 200 on the display 107. In this case, since the second three-dimensional image model is generated without utilizing the first three-dimensional image model, the second three-dimensional image model being generated may be displayed in the area 811, and the two-dimensional image scanned in the second scanning may be displayed in the area 812.

Referring to FIG. 8B, the electronic apparatus 100 may display, on the display 107, a screen 820 including the second three-dimensional image model 821 generated after the second scanning is completed. The generated second three-dimensional image model 821 may be stored in the memory 103.

In addition, the electronic apparatus 100 may display, on the display 107, a screen 820 including an icon 822 indicating aligning of the first three-dimensional image model and the second three-dimensional image model. In particular, in response to the case where the electronic apparatus 100 independently generates the second three-dimensional image model based on only the second scan data values without utilizing the first three-dimensional image model after an input to the button 513 is obtained, the electronic apparatus 100 may display an icon 822 indicating alignment on the display 107. For example, the electronic apparatus 100 may obtain an input to the icon 822 indicating alignment from the user through the input device 109. If the input to the icon 822 indicating alignment is obtained from the user, the electronic apparatus 100 may perform alignment of the first three-dimensional image model and the second three-dimensional image model.

FIGS. 9A to 9E are diagrams illustrating aligning a three-dimensional image model in a first scanning step and a three-dimensional image model in a second scanning step according to an embodiment of the present disclosure. For example, in the case where a three-dimensional image model in the first scanning step is not utilized in the process of generating a three-dimensional image model in the second scanning step, the first three-dimensional image model and the second three-dimensional image model may not be in the aligned state. Therefore, in response to the case where the three-dimensional image model in the first scanning step is not utilized in the process of generating the three-dimensional image model in the second scanning step, a separate alignment process between the three-dimensional image model in the first scanning step and the three-dimensional image model in the second scanning step may be required.

In an embodiment, the electronic apparatus 100 may obtain data indicating at least three points included in the first three-dimensional image model and data indicating at least three points included in the second three-dimensional image model.

In an embodiment, referring to the screen 910 in FIG. 9A, the electronic apparatus 100 may display a first three-dimensional image model 911 and a second three-dimensional image model 912 on the display 107. At this time, the first three-dimensional image model 911 and the second three-dimensional image model 912 displayed on the display 107 may not be in the aligned state.

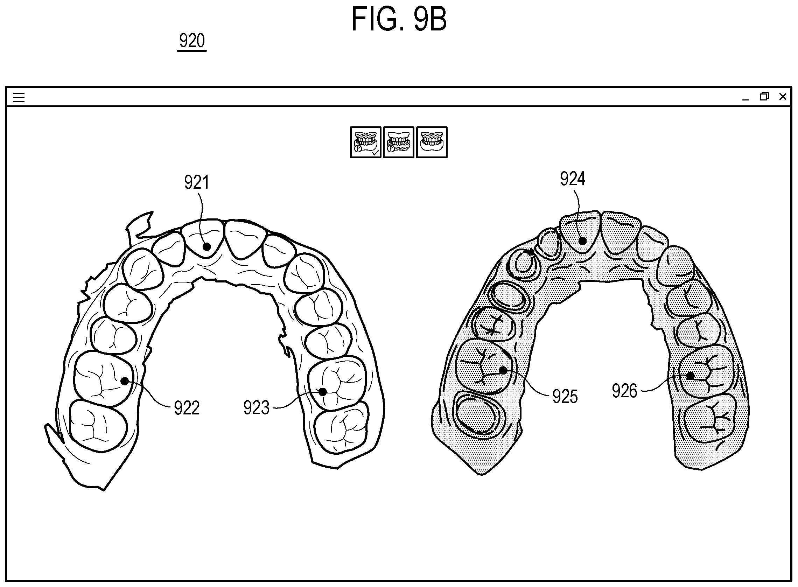

In an embodiment, referring to the screen 920 in FIG. 9B, the electronic apparatus 100 may obtain data indicating at least three teeth included in the first three-dimensional image model and data indicating at least three teeth included in the second three-dimensional image model based on the first three-dimensional image model and second three-dimensional image model displayed on the display 107. For example, the electronic apparatus 100 may obtain inputs of data indicating at least three teeth included in the first three-dimensional image model and data indicating at least three teeth included in the second three-dimensional image model from the user through the input device 109. Subsequently, the electronic apparatus 100 may obtain points corresponding to the at least three teeth for each of the three-dimensional image models. For example, the electronic apparatus 100 may determine points 921, 922, and 923 respectively corresponding to at least three teeth included in the first three-dimensional image model and points 924, 925, and 926 respectively corresponding to at least three teeth included in the second three-dimensional image model. Here, at least three points 921, 922, and 923 included in the first three-dimensional image model may be points indicating, for example, the tooth of number 2, the tooth of number 7, and the tooth of number 13, respectively, when numbered from the left. Similarly, at least three points 924, 925, and 926 included in the second three-dimensional image model may be points indicating, for example, the tooth of number 2, the tooth of number 7, and the tooth of number 13, respectively, when numbered from the left. In another embodiment, the electronic apparatus 100 may analyze each three-dimensional image model, instead of obtaining input of data indicating each tooth from the user, and determine points corresponding to teeth of predetermined numbers (e.g., the tooth of number 2, the tooth of number 7, and the tooth of number 13 from the left).

In an embodiment, obtaining a point corresponding to a specific tooth for each three-dimensional image model may be determined based on an operation of a learned artificial neural network model stored in the memory 103 of the electronic apparatus 100. For example, the learned artificial neural network model may be an artificial neural network model having learned to identify the tooth numbers based on a learning data set including at least one of curvature information, size information, and shape information of each tooth in the three-dimensional image model. For example, the electronic apparatus 100 may determine points, which correspond to the centers of three teeth determined as the tooth of number 2, the tooth of number 7, and the tooth of number 13 by the learned artificial neural network model in the first three-dimensional image model, as at least three points 921, 922, and 923 included in the first three-dimensional image model. In addition, for example, the electronic apparatus 100 may determine, in the second three-dimensional image model, points corresponding to the centers of three teeth determined as the tooth of number 2, the tooth of number 7, and the tooth of number 13 by the learned artificial neural network model as at least three points 924, 925, and 926 included in the second three-dimensional image model. In an embodiment, the tooth of number 2, the tooth of number 7, and the tooth of number 13 are only examples, and points corresponding to teeth of different numbers may be specified according to the embodiment. In addition, one point corresponding to two or more teeth may be specified according to the embodiment. For example, the midpoint of three points corresponding to the respective centers of the tooth of number 12 to the tooth of number 14 may be calculated and determined as the point 926.

In an embodiment, the electronic apparatus 100, based on at least three points included in the first three-dimensional image model and at least three points included in the second three-dimensional image model, may align the second three-dimensional image model with respect to the first three-dimensional image model. On the other hand, the first three-dimensional image model may be aligned with respect to the second three-dimensional image model, or both three-dimensional image models may be aligned simultaneously.

In another embodiment, even if the first scan data values for the entire oral cavity are obtained in the first scanning and if the second scan data values for a part of the oral cavity are obtained in the second scanning, the electronic apparatus 100 may align the first three-dimensional image model and the second three-dimensional image model. In this case, the electronic apparatus 100 may preferentially identify that only a part of the oral cavity is scanned in the second scanning and that the entire oral cavity is scanned in the first scanning. The electronic apparatus 100 may collect the second scan data values and the first scan data values for another part of the oral cavity, which are not obtained in the second scanning, and generate a second three-dimensional image model for the entire oral cavity. Subsequently, the electronic apparatus 100 may align at least one of the generated second three-dimensional image model and first three-dimensional image model. In an embodiment, although an example in which the entire oral cavity is scanned in the first scanning and in which a part of the oral cavity is scanned in the second scanning has been described, on the other hand, in the case where the entire oral cavity is scanned in the second scanning and where a part of the oral cavity is scanned in the first scanning, the electronic apparatus 100 may regenerate the first three-dimensional image model using some of the second scan data values and the first scan data values and then align at least one of the first three-dimensional image model and the second three-dimensional image model.

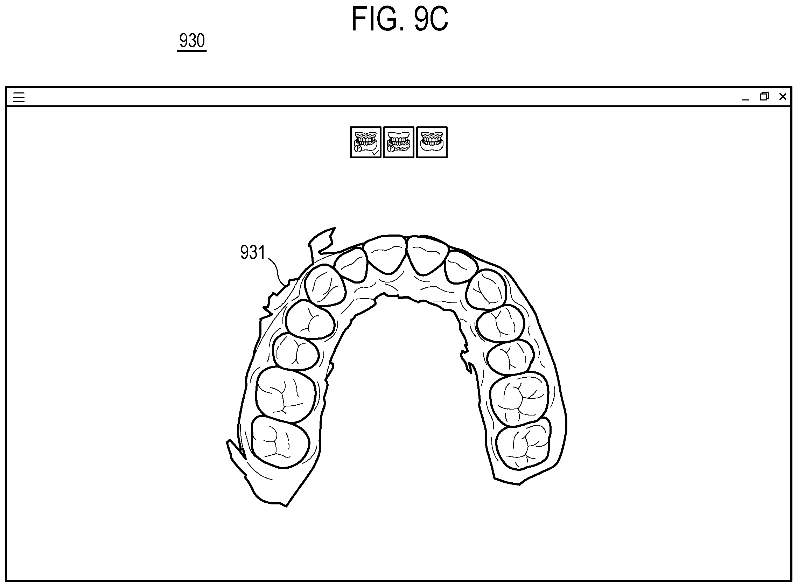

The electronic apparatus 100 may perform a transformation, such as a translation transformation or a rotation transformation, on the first three-dimensional image model and/or the second three-dimensional image model such that a plane including coordinate values corresponding to at least three points 921, 922, and 923 included in the first three-dimensional image model and a plane including coordinate values corresponding to at least three points 924, 925, and 926 included in the second three-dimensional image model match each other. In addition, the electronic apparatus 100 may perform a transformation, such as a translation transformation, on the first three-dimensional image model and/or the second three-dimensional image model such that a reference point of the first three-dimensional image model and a reference point of the second three-dimensional image model match each other. As a result, referring to the screen 930 in FIG. 9C, the electronic apparatus 100 may generate a first synthetic image model 931 in which the first three-dimensional image model and the second three-dimensional image model, which have been aligned, are superimposed and display the first synthetic image model 931 on the display 107. At this time, if the first three-dimensional image model and the second three-dimensional image model are superimposed, a three-dimensional image model that is substantially identical to the first three-dimensional image model before the tooth is cut may be displayed on the display 107.

In an embodiment, the electronic apparatus 100 may process the first three-dimensional image model in the first synthetic image model 931 to be transparent or translucent. For example, the electronic apparatus 100 may obtain an input for displaying the second three-dimensional image model of the prep scanning step from the user through the input device 109. For example, the electronic apparatus 100 may display an icon (not shown) for instructing to display the second three-dimensional image model on the screen 930. In addition, the electronic apparatus 100 may obtain an input for selecting the icon (not shown) for instructing to display the second three-dimensional image model. In another embodiment, the first three-dimensional image model may be processed to be transparent or translucent in the first synthetic image model 931 by default settings or settings input in advance by the user. If the first three-dimensional image model is processed to be transparent or translucent in the first synthetic image model 931, the second three-dimensional image model may be displayed at least faintly on the screen 930, and the user may compare the first three-dimensional image model and the second three-dimensional image model to identify both images.

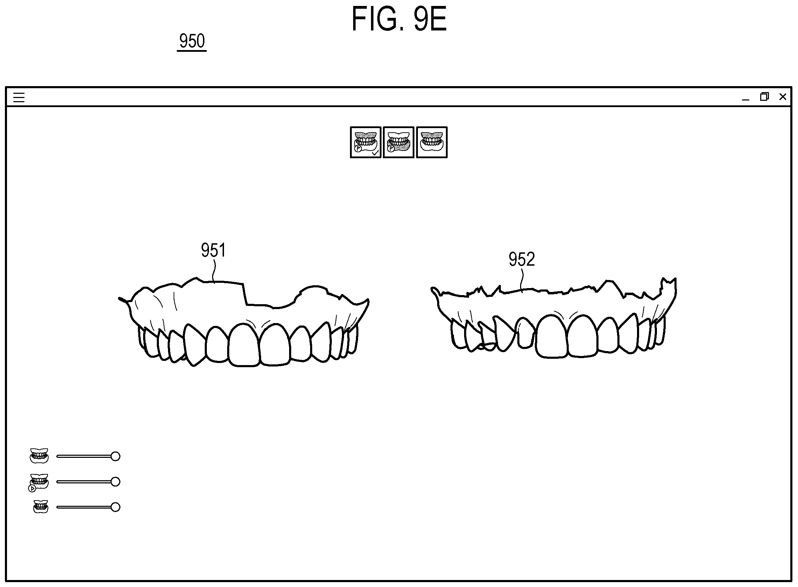

In an embodiment, if the first three-dimensional image model and the second three-dimensional image model are not aligned and the corresponding scanning step (i.e., the second scanning step) is about to end although the second three-dimensional image model is generated in the second scanning step without utilizing the first three-dimensional image model in the first scanning step, the electronic apparatus 100 may output a pop-up message 941 on the screen 940. The output pop-up message 941 may include a button 942 for instructing to cancel the end of the corresponding scanning step, a button 943 for instructing to skip the alignment of the first three-dimensional image model and the second three-dimensional image model, and a button 944 for executing the alignment of the first three-dimensional image model and the second three-dimensional image model. For example, if an input to the button 942 is obtained, the electronic apparatus 100 may cancel the end of the corresponding scanning step and continue the corresponding scanning step. Additionally, if an input to the button 943 is obtained, the electronic apparatus 100 may not align the first three-dimensional image model and the second three-dimensional image model. In this case, as shown in the screen 950 in FIG. 9E, the electronic apparatus 100 may display the first three-dimensional image model 951 and the second three-dimensional image model 952, which are not aligned, on the display 107. In addition, if an input to the button 944 is obtained, the electronic apparatus 100 may align the first three-dimensional image model and the second three-dimensional image model. In this case, the electronic apparatus 100 may display the screen 910 in FIG. 9A on the display 107.

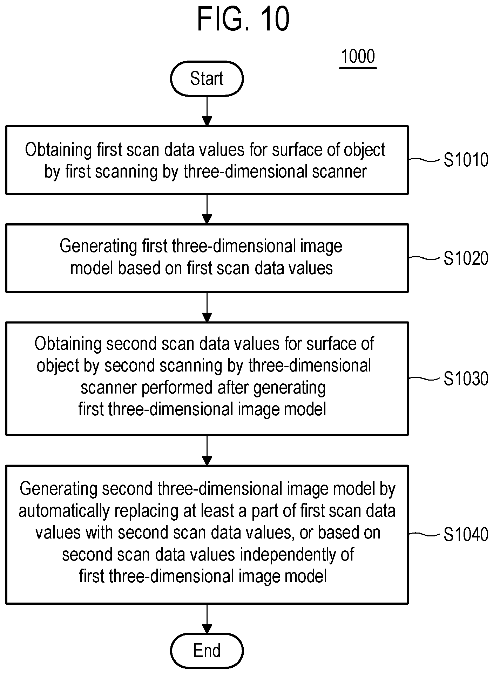

FIG. 10 is a flowchart illustrating a scan image processing method 1000 of a three-dimensional scanner according to an embodiment of the present disclosure. The scan image processing method 1000 of the three-dimensional scanner may be performed by the electronic apparatus 100. Referring to FIG. 10, the scan image processing method 1000 of the three-dimensional scanner may include an operation S1010 of obtaining first scan data values for a surface of an object by first scanning by the three-dimensional scanner, an operation S1020 of generating a first three-dimensional image model based on the first scan data values, an operation S1030 of obtaining second scan data values for the surface of the object by second scanning by the three-dimensional scanner performed after generation of the first three-dimensional image model, and an operation S1040 of generating a second three-dimensional image model by automatically replacing at least a part of the first scan data values with the second scan data values or based on the second scan data values, independently of the first three-dimensional image model.

In an embodiment, operation S1040 may include an operation of comparing the first scan data values with the second scan data values to determine first sub-data values that are at least a part of the first scan data values and second sub-data values that are at least a part of the second scan data values, and an operation of generating the second three-dimensional image model based on third scan data values obtained by replacing the first sub-data values of the first scan data values with the second sub-data values. In an embodiment, the operation of determining the first sub-data values and the second sub-data value may include an operation of determining first vectors connecting a virtual focus of the three-dimensional scanner and the first scan data values or second vectors connecting the virtual focus and the second scan data values, an operation of determining whether or not the first vectors intersect the second scan data values or whether or not the second vectors intersect the first scan data values, and an operation of, if at least one first vector among the first vectors intersects at least one second scan data value among the second scan data values, determining data values of the first scan data values, which are associated with the at least one first vector intersecting the at least one second scan data value, as the first sub-data values and determining data values of the second scan data values, which intersect the at least one first vector, as the second sub-data values, and if at least one second vector among the second vectors intersects at least one first scan data value among the first scan data values, determining data values of the first scan data values, which intersect the at least one second vector, as the first sub-data values and determining data values of the second scan data values, which are associated with the at least one second vector intersecting the at least one first scan data value, as the second sub-data values.

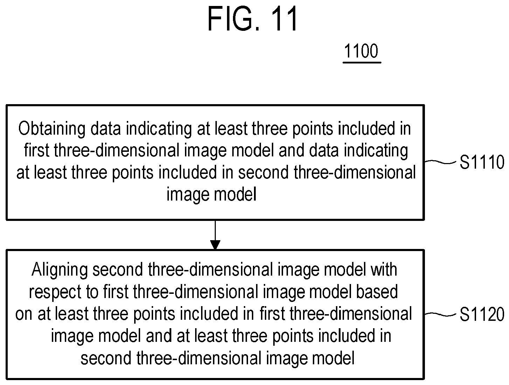

FIG. 11 is a flowchart illustrating an operation 1100 of aligning a three-dimensional image model in a first scanning step and a three-dimensional image model in a second scanning step according to an embodiment of the present disclosure. Referring to FIG. 11, the operation 1100 may include an operation S1110 of obtaining data indicating at least three points included in the first three-dimensional image model and data indicating at least three points included in the second three-dimensional image model, and an operation S1120 of aligning the first three-dimensional image model or the second three-dimensional image model based on the at least three points included in the first three-dimensional image model and the at least three points included in the second three-dimensional image model.

In an embodiment, the operation S1110 may include an operation of displaying the first three-dimensional image model and the second three-dimensional image model on the display, an operation of obtaining inputs of data indicating at least three teeth included in the first three-dimensional image model and data indicating at least three teeth included in the second three-dimensional image model based on the first three-dimensional image model and the second three-dimensional image model displayed on the display, and an operation of determining three points respectively corresponding to the at least three teeth included in the first three-dimensional image model and three points respectively corresponding to the at least three teeth included in the second three-dimensional image model.

In an embodiment, the operation S1100 may further include an operation of generating a first synthetic image model in which the first three-dimensional image model and the second three-dimensional image model, which have been aligned, are superimposed, and an operation of displaying the first synthetic image model on the display. In addition, in an embodiment, the operation of displaying the first synthetic image model may include an operation of obtaining a third signal, and an operation of processing the first three-dimensional image model to be transparent or translucent in the first synthetic image model.

In the flowcharts according to the present disclosure, although the respective steps of the method or algorithm are described in sequence, the respective steps may be performed in any combination order, in addition to the sequential execution. The descriptions of the flowcharts of the present disclosure do not exclude changes or modifications to the method or algorithm, and do not imply that any step is essential or desirable. In an embodiment, at least a part of the steps may be performed in parallel, iteratively, or heuristically. In another embodiment, at least a part of the steps may be omitted, or other steps may be added thereto.

The embodiments according to the present disclosure may be implemented as software on a machine-readable storage medium. The software may be software for implementing the embodiments described in the present disclosure. The software may be inferred from the embodiments described in the present disclosure by programmers in the art to which the present disclosure belongs. For example, the software may be a program including machine-readable instructions (e.g., codes or code segments). The machine is a device capable of operating according to instructions called from a storage medium, and may be, for example, a computer. In an embodiment, the machine may be a computing device according to the embodiments described in the present disclosure. In an embodiment, the processor of the machine may execute the called instructions to cause the components of the machine to perform functions corresponding to the instructions. The storage medium may indicate any kind of recording medium capable of being read by the machine and storing data. The storage medium may include, for example, a ROM, a RAM, a CD-ROM, a magnetic tape, a floppy disk, an optical data storage device, or the like. In an embodiment, the storage medium may be implemented in a distributed form in a computer system connected to a network. In this case, the software may be distributed, stored, and executed in the computer system or the like. In another embodiment, the storage medium may be a non-transitory storage medium. A non-transitory storage medium indicates a tangible medium regardless of whether data is stored semi-permanently or temporarily, and does not include a signal that is transitorily transmitted.

Although the technical idea according to the present disclosure has been described in the above embodiments, the technical idea according to the present disclosure includes various substitutions, modifications, and changes capable of being made within the scope that those skilled in the art to which the present disclosure belongs may understand. In addition, it should be understood that such substitutions, modifications, and changes may be included within the scope of the appended claims.

## Claims

1. An electronic apparatus comprising:
a communication circuit in communication connection with a three-dimensional scanner;
one or more processors; and
one or more memories storing instructions executed by the one or more processors, wherein, when the instructions are executed by the one or more processors, the one or more processors:
obtain first scan data values for a surface of an object by first scanning by the three-dimensional scanner, the first scan data values including three-dimensional coordinate values;
generate a first three-dimensional image model based on the first scan data values;
obtain second scan data values for the surface of the object by second scanning by the three-dimensional scanner performed after generation of the first three-dimensional image model, the second scan data values including three-dimensional coordinate values; and
generate a second three-dimensional image model by automatically replacing at least a part of the first scan data values with the second scan data values or based on the second scan data values independently of the first three-dimensional image model.

2. The electronic apparatus of Claim 1, wherein the generating of the second three-dimensional image model by automatically replacing at least a part of the first scan data values with the second scan data values includes:
comparing the first scan data values with the second scan data values to determine first sub-data values that are at least a part of the first scan data values and second sub-data values that are at least a part of the second scan data values; and
generating the second three-dimensional image model based on third scan data values obtained by replacing the first sub-data values of the first scan data values with the second sub-data values.

3. The electronic apparatus of Claim 2, wherein the determining of the first sub-data values and the second sub-data value includes:
determining first vectors connecting a virtual focus of the three-dimensional scanner and the first scan data values or second vectors connecting the virtual focus and the second scan data values;
determining whether or not the first vectors intersect the second scan data values or whether or not the second vectors intersect the first scan data values;
when at least one first vector among the first vectors intersects at least one second scan data value among the second scan data values, determining data values of the first scan data values, which are associated with the at least one first vector intersecting the at least one second scan data value, as the first sub-data values and determining data values of the second scan data values, which intersect the at least one first vector, as the second sub-data values; and
when at least one second vector among the second vectors intersects at least one first scan data value among the first scan data values, determining data values of the first scan data values, which intersect the at least one second vector, as the first sub-data values and determining data values of the second scan data values, which are associated with the at least one second vector intersecting the at least one first scan data value, as the second sub-data values.

4. The electronic apparatus of Claim 1, wherein the one or more processors are configured to, after generating the second three-dimensional image model based on the second scan data value:
obtain data indicating at least three points included in the first three-dimensional image model and data indicating at least three points included in the second three-dimensional image model; and
align the first three-dimensional image model or the second three-dimensional image model based on the at least three points included in the first three-dimensional image model and the at least three points included in the second three-dimensional image model.

5. The electronic apparatus of Claim 4, further comprising a display,
wherein the one or more processors are configured to:
display the first three-dimensional image model and the second three-dimensional image model on the display;
obtain inputs of data indicating at least three teeth included in the first three-dimensional image model and data indicating at least three teeth included in the second three-dimensional image model based on the first three-dimensional image model and the second three-dimensional image model displayed on the display; and
determine three points respectively corresponding to the at least three teeth included in the first three-dimensional image model and three points respectively corresponding to the at least three teeth included in the second three-dimensional image model.

6. The electronic apparatus of Claim 4, further comprising a display,
wherein the one or more processors are configured to:
generate a first synthetic image model in which the first three-dimensional image model and the second three-dimensional image model, which have been aligned, are superimposed; and
display the first synthetic image model on the display.

7. The electronic apparatus of Claim 6, wherein the one or more processors are configured to process the first three-dimensional image model to be transparent or translucent in the first synthetic image model.

8. A method for processing a scan image obtained by a three-dimensional scanner performed by an electronic apparatus including one or more processors and one or more memories storing instructions to be executed by the one or more processors, the method comprising:
obtaining first scan data values for a surface of an object by first scanning by the three-dimensional scanner, the first scan data values comprising three-dimensional coordinate values;
generating a first three-dimensional image model based on the first scan data values;
obtaining second scan data values for the surface of the object by second scanning by the three-dimensional scanner performed after generation of the first three-dimensional image model, the second scan data values comprising three-dimensional coordinate values; and
generating a second three-dimensional image model by automatically replacing at least a part of the first scan data values with the second scan data values or based on the second scan data values, independently of the first three-dimensional image model.

9. The method of Claim 8, wherein the generating of the second three-dimensional image model by automatically replacing at least a part of the first scan data values with the second scan data values includes:
comparing the first scan data values with the second scan data values to determine first sub-data values that are at least a part of the first scan data values and second sub-data values that are at least a part of the second scan data values; and
generating the second three-dimensional image model based on third scan data values obtained by replacing the first sub-data values of the first scan data values with the second sub-data values.

10. The method of Claim 9, wherein the determining of the first sub-data values and the second sub-data value includes:
determining first vectors connecting a virtual focus of the three-dimensional scanner and the first scan data values or second vectors connecting the virtual focus and the second scan data values;
determining whether or not the first vectors intersect the second scan data values or whether or not the second vectors intersect the first scan data values;
when at least one first vector among the first vectors intersects at least one second scan data value among the second scan data values, determining data values of the first scan data values, which are associated with the at least one first vector intersecting the at least one second scan data value, as the first sub-data values and determining data values of the second scan data values, which intersect the at least one first vector, as the second sub-data values; and
when at least one second vector among the second vectors intersects at least one first scan data value among the first scan data values, determining data values of the first scan data values, which intersect the at least one second vector, as the first sub-data values and determining data values of the second scan data values, which are associated with the at least one second vector intersecting the at least one first scan data value, as the second sub-data values.

11. The method of Claim 8, further comprising, after generating the second three-dimensional image model based on the second scan data value, independently of the first three-dimensional image model:
obtaining data indicating at least three points included in the first three-dimensional image model and data indicating at least three points included in the second three-dimensional image model; and
aligning the first three-dimensional image model or the second three-dimensional image model based on the at least three points included in the first three-dimensional image model and the at least three points included in the second three-dimensional image model.

12. The method of Claim 11, wherein the obtaining of data indicating at least three points included in the first three-dimensional image model and data indicating at least three points included in the second three-dimensional image model includes:
displaying the first three-dimensional image model and the second three-dimensional image model on a display;
obtaining inputs of data indicating at least three teeth included in the first three-dimensional image model and data indicating at least three teeth included in the second three-dimensional image model based on the first three-dimensional image model and the second three-dimensional image model displayed on the display; and
determining three points respectively corresponding to the at least three teeth included in the first three-dimensional image model and three points respectively corresponding to the at least three teeth included in the second three-dimensional image model.

13. The method of Claim 11, further comprising:
generating a first synthetic image model in which the first three-dimensional image model and the second three-dimensional image model, which have been aligned, are superimposed; and
displaying the first synthetic image model on a display.

14. The method of Claim 13, further comprising
processing the first three-dimensional image model to be transparent or translucent in the first synthetic image model.

15. A non-transitory computer-readable recording medium storing instructions executable by one or more processors,
wherein, when executed by the one or more processors, the instructions cause the one or more processors to:
obtain first scan data values for a surface of an object by first scanning by the three-dimensional scanner, the first scan data values comprising three-dimensional coordinate values;
generate a first three-dimensional image model based on the first scan data values;
obtain second scan data values for the surface of the object by second scanning by the three-dimensional scanner performed after generation of the first three-dimensional image model, the second scan data values comprising three-dimensional coordinate values; and
generate a second three-dimensional image model by automatically replacing at least a part of the first scan data values with the second scan data values or based on the second scan data values, independently of the first three-dimensional image model.
